Europäisches Patentamt

European Patent Office

Office européen des brevets

(11) Veröffentlichungsnummer: **0 276 370 A2**

(12) # EUROPÄISCHE PATENTANMELDUNG

(21) Anmeldenummer: 87114497.8

(22) Anmeldetag: 05.10.87

(51) Int. Cl.⁴: **A61K 31/725**

(30) Priorität: 09.10.86 DE 3634392

(43) Veröffentlichungstag der Anmeldung:
03.08.88 Patentblatt 88/31

(84) Benannte Vertragsstaaten:
**AT BE CH DE FR GB IT LI NL SE**

(71) Anmelder: **KNOLL AG**
**Knollstrasse**
**D-6700 Ludwigshafen(DE)**

(72) Erfinder: **Herr, Dieter, Dr.**
**Hardenburgstrasse 19**
**D-6701 Altrip(DE)**
Erfinder: **Müller, Claus D., Dr.**
**Im Schafläger 30**
**D-6806 Viernheim(DE)**
Erfinder: **Betz, Eberhard, Prof., Dr.**
**Sudetenstrasse 41**
**D-7400 Tübingen(DE)**
Erfinder: **Hämmerle, Hugo**
**Lange Furche 35**
**D-7400 Tübingen(DE)**

(74) Vertreter: **Karau, Wolfgang Dr.**
**BASF Aktiengesellschaft Carl-Bosch-Strasse 38**
**D-6700 Ludwigshafen(DE)**

(54) Verwendung niedermolekularer Dextransulfate als Arteriosklerosemittel.

(57) Es wird die Verwendung von niedermolekularem Dextransulfat bei der Bekämpfung von Arteriosklerose beschrieben.

EP 0 276 370 A2

## Verwendung polysulfatierter niedermolekularer Dextransulfate

Die Erfindung betrifft eine neue Verwendung von niedermolekularen Dextransulfaten.

Niedermolekulare Dextransulfate sowie deren Verwendung als Antikoagulantien sind bereits aus der US-PS 2 715 091 bekannt.

Gegenstand der Erfindung ist die Verwendung von niedermolekularem Dextransulfat und dessen Salzen mit physiologisch verträglichen Basen bei der Behandlung der Arteriosklerose und/oder zur Initiierung einer Angiogenese bzw. zur Herstellung von Arzneimitteln zur Behandlung von Arteriosklerose und/oder zur Initiierung einer Angionese.

Die erfindungsgemäß zu verwendenden Dextransulfate besitzen ein Molekulargewicht von etwa 1.500 bis 20.000 Dalton, vorzugsweise 2.000 bis 9.000 Dalton und einen Schwefelgehalt von 0,5 bis 20, vorzugsweise 7 bis 17 und insbesondere 7 bis 8 Gew.%. Sie werden hergestellt durch Sulfatierung der entsprechenden Dextrane, vgl. US-PS 2 715 091.

Zur Salzbildung eignen sich physiologisch verträgliche Basen wie Alkali-und Erdalkalihydroxide und insbesondere Natrium-und Calciumhydroxid.

Mit den erfindungsgemäßen Verbindungen kann die Bildung von Atheromen in der Gefäßwand und die Progression bereits bestehender arteriosklerotischer Veränderungen gehemmt bzw. deren Regression gefördert werden. Darüberhinaus kann durch eine Förderung der Proliferation von Endothelzellen die Angiogenese angeregt und die Gefäßversorgung von Organen, deren Durchblutung z.B. durch arteriosklerotische Prozesse reduziert ist, verbessert werden. Ferner fördern die neuen Verbindungen die Endothelialisierung nach chirurgischem Gefäßersatz.

Zum Nachweis dieser Wirkungen wurden Kulturen von glatten arteriellen Gefäßmuskelzellen (SMC) und von arteriellen Endothelzellen (EC) mit den Substanzen in einer Konzentration von $5 \times 10^{-5}$ M inkubiert. Bestimmt wurde der Zuwachs an Zellen innerhalb von 7 Tagen nach Substanzzugabe. Als Maß für die Beeinflussung der Proliferation (Hemmung, Stimulation) durch die Prüfsubstanzen diente die relative Änderung (%) im Vergleich zu unbehandelten Kontrollkulturen.

Ferner wurde der Einfluß der Verbindungen auf das Migrationsverhalten von SMC und EC bestimmt. Dazu wurden die Zellen in Kulturschalen gezüchtet, bis sich ein Multilayer (SMC) bzw. Monolayer (EC) gebildet hatte. Im Anschluß daran wurden die Kulturen 48 Stunden lang mit den zu untersuchenden Substanzen inkubiert. Danach wurde mit einer Rasierklinge der Zellrasen so angeschnitten, daß ein scharfer Rand und eine freie Fläche entstand, in die die Zellen einwandern können. Die migrierten Zellen wurden nach weiteren 72 Stunden ausgezählt. Als Maß für die Beeinflussung der Migration durch die Prüfsubstanzen diente die relative Änderung der Zahl der migrierten Zellen (%) im Vergleich zu unbehandelten Kontrollkulturen.

Die blutgerinnungshemmende Wirksamkeit der neuen Verbindungen wurde über die aktivierte partielle Thromboplastinzeit (APTT) ("Kleiner Gerinnungsstatus", Behringwerke Marburg) und über die Hemmung des Gerinnungsfaktors Xa (Throm. Res. 10, 399, 1977) an Humanplasma bestimmt. Als Vergleichssubstanz diente Heparin-Na.

Ein bedeutender Faktor bei der Pathogenese arteriosklerotischer Gefäßveränderungen ist die Migration und die Proliferation glatter Gefäßmuskelzellen (SMC). Natürliche Heparine sind außer der bekannten Blutgerinnungshemmung auch in der Lage, die Proliferation und die Migration von vaskulären SMC zu hemmen und dadurch der Atherogenese entgegenzuwirken (A.W. Clowes and M.J. Karnovsky, Nature, 265, 625, 1977).

Bei den erfindungsgemäßen Substanzen steht diese Proliferation-resp. Migrationshemmung im Vordergrund der Wirkung (Tab. I). Die für die antiatherogene Wirkung unerwünschte blutgerinnungshemmende Wirkung ist wesentlich schwächer ausgebildet. Die Verbindungen wirken somit selektiv antiatherogen. Gemessen an der Wirkung des zum Vergleich mitgeführten Heparins ist bei stärkerer (Substanzen 11, 12) oder etwa gleich starker (Substanzen 16, 17) antiproliferativer Wirksamkeit an SMC die gerinnungshemmende Wirkung (12-24 mal (APTT) bzw. 530-1550 mal (Anti-Xa-Aktivität) geringer als die der Vergleichssubstanz.

Auf die Proliferation arterieller Endothelzellen haben die erfindungsgemäßen Substanzen wie Heparin-Na keinen Einfluß oder sie sind diesem durch eine die Proliferation stimulierende Komponente überlegen (Substanzen 16, 17).

Die Migration von EC wird durch die neuen Verbindungen weniger stark gehemmt als durch Heparin-Na. Eine Ausnahme bildet die Substanz 16, das außer der Proliferation (s.o.) auch die Migration der EC steigert.

Durch die proliferations-bzw. migrationssteigernde Wirkung der Substanzen 16 und 17 kann die

2

Neubildung von Gefäßen und die Reparatur von Endothelschäden bzw. die Endothelialisierung nach chirurgischem Gefäßersatz gefördert werden.

Tabelle I

| Substanz | Antiatherogene Wirkung | | | | Hemmung der Blutgerinnung | |
|---|---|---|---|---|---|---|
| | Änderung der Proliferation (%) | | Änderung der Migration (%) | | APTT[3] IE/Mg | Anti-Xa-Aktivität IE/Mg |
| | SMC[1] | EC[2] | SMC | EC | | |
| Substanz 11 | - 40 | 0 | - 40 | - 10 | 10 | 0,11 |
| Substanz 12 | - 40 | 0 | - 40 | - 13 | 14 | 0,30 |
| Substanz 16 | - 30 | - 20 | - 30 | + 25 | 8,6 | 0,16 |
| Substanz 17 | - 30 | + 40 | - 30 | - 40 | 6,6 | 0,16 |
| Natürliches Heparin | - 28 | 0 | - 40 | - 45 | 160 | 160 |

[1] SMC = glatte Muskelzelle

[2] EC = Endothelzelle

[3] APTT = aktivierte partielle Thromboplastinzeit

Die Dextransulfate werden vorzugsweise parenteral in Mengen von normalerweise zwischen etwa 3 und 30 mg pro Patient und Tag verabfolgt.

Herstellung von polysulfatierten Dextranen

Zur Herstellung der niedermolekularen polysulfatierten Dextrane wurden als Ausgangssubstanzen Dextran 1 (Molekulargewicht 1000 Dalton), Dextran 2 (Molekulargewicht 2000 Dalton), Dextran 4 (Molekulargewicht 4000 Dalton), Dextran 8 (Molekulargewicht 6000 bis 8000 Dalton) und Dextran 15 (Molekulargewicht 12.000 bis 15.000 Dalton) verwendet.

Beispiel 1

Polysulfatierung von Dextran 1

20 g Dextran 1 wurden mit 500 ml Pyridin/Dimethylformamid (1:1) unter Einleitung von Stickstoff bei 55°C suspendiert. Unterschiedliche Mengen (zwischen 5 und 60 ml) Chlorsulfonsäure wurden unter Rühren bei 85°C in 300 ml Pyridin eingetropft. Nach kurzem Aufrühren wurde die Pyridin-Chlorsulfonsäuremischung in die vorgelegte Dextransuspension eingebracht und 30 min bei 75°C nachgerührt. Danach wurde das Gemisch auf 25°C im Eisbad abgekühlt. Es entstand ein öliger Niederschlag, der mehrmals mit Pyridin/Dimethylformamid nachgewaschen wurde. Dieser Niederschlag wurde in 100 ml 0,5 %iger Natriumchloridlösung gelöst und mit den 6fachen Volumen an Ethanol gefällt. Das Sediment wurde in destilliertem Wasser gelöst und über einen Kationenaustauscher, Typ DOWEX® MSC-1, von anhaftenden Pyridin-Spuren befreit. Die kationenfreie Dextransulfatlösung wurde anschließend mit 10 N NaOH auf pH 7 eingestellt und nochmals mit den 6fachen Volumen an Methanol gefällt. Das Sediment wurde in destilliertem Wasser aufgenommen, filtriert und gefriergetrocknet.

Der Sulfatierungsgrad der so erhaltenen Produkte variiert stark in Abhängigkeit von der eingesetzten Menge Chlorsulfonsäure. Die folgende Tabelle gibt eine Übersicht über die erhaltenen Produkte.

| Substanz Nr. | Eingesetztes Dextran | Verwendete Menge Chlorsulfonsäure (ml) pro 20 g Dextran | Schwefelgehalt des Produktes (%) | M (Dalton) |
|---|---|---|---|---|
| 1 | Dextran 1 | 5 | 4,5 | 1.400 |
| 2 | Dextran 1 | 15 | 7,8 | 1.800 |
| 3 | Dextran 1 | 20 | 13,1 | 2.400 |
| 4 | Dextran 1 | 30 | 16,8 | 2.600 |
| 5 | Dextran 1 | 50 | 19,7 | 3.100 |
| 6 | Dextran 2 | 10 | 7,2 | 2.400 |
| 7 | Dextran 2 | 20 | 14,1 | 2.800 |
| 8 | Dextran 2 | 50 | 18,1 | 3.600 |
| 9 | Dextran 4 | 10 | 5,2 | 4.200 |
| 10 | Dextran 4 | 20 | 9,8 | 4.400 |
| 11 | Dextran 4 | 40 | 14,1 | 4.800 |
| 12 | Dextran 4 | 50 | 16,5 | 5.100 |
| 13 | Dextran 8 | 20 | 7,8 | 7.700 |
| 14 | Dextran 8 | 40 | 14,8 | 8.500 |
| 15 | Dextran 8 | 60 | 17,4 | 9.400 |
| 16 | Dextran 15 | 20 | 7,6 | 15.800 |
| 17 | Dextran 15 | 30 | 9,4 | 9.000 |
| 18 | Dextran 15 | 40 | 15,2 | 16.200 |
| 19 | Dextran 15 | 60 | 18,4 | 19.100 |

Galenisches Beispiel

10 g Dextran 2 (Schwefelgehalt 7,2 %) werden in 5000 ml Wasser unter Zusatz von NaCl gelöst und mit 0,1 N NaOH auf pH 6,0 eingestellt, so daß eine blutisotonische Lösung entsteht. Jeweils 5 ml dieser Lösung werden in Ampullen gefüllt und sterilisiert.

**Ansprüche**

1. Verwendung von niedermolekularem Dextransulfat und dessen Salzen mit physiologisch verträglichen Basen bei der Behandlung von Arteriosklerose und/oder zur Initiierung einer Angiogenese.

2. Verwendung von niedermolekularem Dextransulfat und dessen Salzen mit physiologisch verträglichen Basen zur Herstellung von Arzneimitteln zur Behandlung von Arteriosklerose und/oder zur Initiierung einer Angiogenese.